(19) **Europäisches Patentamt**
**European Patent Office**
**Office européen des brevets**

(11) **EP 3 070 507 B1**

(12) **EUROPEAN PATENT SPECIFICATION**

(45) Date of publication and mention
of the grant of the patent:
**15.05.2019 Bulletin 2019/20**

(21) Application number: **14861990.1**

(22) Date of filing: **10.11.2014**

(51) Int Cl.:
***A61B 1/00*** *(2006.01)*　　***A61B 1/05*** *(2006.01)*
***G02B 23/24*** *(2006.01)*　　***G03B 17/17*** *(2006.01)*
***G03B 35/08*** *(2006.01)*　　***G03B 37/00*** *(2006.01)*

(86) International application number:
**PCT/JP2014/079736**

(87) International publication number:
**WO 2015/072427 (21.05.2015 Gazette 2015/20)**

(54) **ENDOSCOPE IMAGING DEVICE**

**ENDOSKOPBILDGEBUNGSVORRICHTUNG**

**DISPOSITIF D'IMAGERIE ENDOSCOPIQUE**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.11.2013 JP 2013235948**

(43) Date of publication of application:
**21.09.2016 Bulletin 2016/38**

(73) Proprietor: **Olympus Corporation
Tokyo 192-8507 (JP)**

(72) Inventors:
 • **NAMII, Yasushi
  Hachioji-shi
  Tokyo 192-8507 (JP)**

 • **NAGAOKA, Hideyuki
  Hachioji-shi
  Tokyo 192-8507 (JP)**

(74) Representative: **Schicker, Silvia
Wuesthoff & Wuesthoff
Patentanwälte PartG mbB
Schweigerstraße 2
81541 München (DE)**

(56) References cited:
 **JP-A- H05 346 556　　JP-A- H10 239 594
 JP-A- 2010 056 345　　US-A1- 2003 071 271
 US-A1- 2009 079 019　　US-A1- 2010 059 844
 US-B1- 6 476 851**

## Description

{Technical Field}

The present invention relates to an image acquisition device and particularly to an endoscope image-acquisition device to be applied to an endoscope with which a specimen under test can be stereoscopically observed.

{Background Art}

In an image acquisition device to be applied to an endoscope that allows stereoscopic viewing, an image is acquired by using one imaging element for two objective optical systems in order to stereoscopically image a specimen under test; therefore, light obliquely enters the imaging element from the subject.

As a measure against shading caused by oblique incidence of an objective optical system, for example, PTL 1 discloses a technique in which the positions of microlenses in an imaging element are shifted with respect to light-sensitive portions in directions away from the center portion of the imaging element toward circumferential portions thereof, according to the characteristics of oblique incidence of the objective optical system.

PTL 2 points out a reduction in color reproducibility due to color mixing and a reduction in resolving power, which are caused by crosstalk that occurs between pixels in an image sensor, and discloses a technique in which microlenses are shifted with respect to the center positions of photodiodes, as in PTL 1, in particular, in order to suppress crosstalk due to light rays having oblique incident angles.

PTL 3 discloses a technique in which the positions of microlenses are shifted according to the characteristics of oblique incidence from the center portion of each objective optical system, thus reducing shading and making it possible to suppress crosstalk at the same time. PTL 3 describes an electronic endoscope having an objective system constructed with an objective lens system for side view, an objective lens system for direct view, and a CCD. The objective lens systems and are each corrected for distortion in such a way that a chief ray directed toward the maximum image height is inclined outwardly with respect to the optical axis. For the entrance surface of the CCD, in separating from the optical axis of the objective lens systems, a shift between the optical axis of each microlens and the center of each color filter and photosensor arranged opposite thereto is progressively increased away from the optical axis of the objective lens system.

{Citation List}

{Patent Literature}

{PTL 1} Japanese Unexamined Patent Application, Publication No. Hei 05-346556
{PTL 2} Japanese Unexamined Patent Application, Publication No. 2010-56345
{PTL 3} Publication of Japanese Patent No. 4054094

{Summary of Invention}

{Technical Problem}

However, in the imaging element in PTL 3, the positions of the microlenses are determined by assuming an image acquisition device having two objective optical systems; therefore, the imaging element in PTL 3 cannot be applied to an image acquisition device having one objective optical system. In other words, an imaging element that is fitted into an image acquisition device having one objective optical system cannot be directly applied to an image acquisition device having two objective optical systems. Thus, it is necessary to manufacture imaging elements for respective image acquisition devices, thus increasing the manufacturing costs of imaging elements.

Incidentally, when a telecentric objective optical system is used to form two images arrayed in parallel on an imaging element in which there are no shifts between microlenses and light-sensitive portions of the imaging element, conforming to the telecentric optical system, shading does not occur. However, if such a telecentric optical system is applied, the objective optical system is increased in size, thus making it difficult to array such objective optical systems in parallel.

The present invention has been made in view of the above-described circumstances, and an object thereof is to provide an endoscope image-acquisition device that is capable of acquiring a plurality of images arrayed in parallel while suppressing shading and crosstalk, that eliminates the need to manufacture imaging elements for respective image acquisition devices, and that allows a size reduction.

{Solution to Problem}

In order to achieve the above-described object, the present invention provides an endoscope image-acquisition device having the features of claim 1. Preferred embodiments are defined by the dependent claims. Any example or embodiment which does not fall under the scope of the independent claims is not part of the invention.
According to the present invention, the centers of the light-sensitive portions at the pixels in the imaging element are displaced with respect to the optical axes of the microlenses such that the displacements therebetween are gradually increased in peripheral directions away from the center portion of the imaging element toward peripheral portions thereof; the position of the exit pupil of the objective optical system is closer to an object than the imaging position of the objective optical system is; and the above-described conditional expression (1) is satisfied. Even though an imaging element that is fitted into an image acquisition device that forms one image in the entire imaging range is directly applied to an image acquisition device that forms two images arrayed in parallel, it is possible to realize a compact image acquisition device while suppressing shading and crosstalk. Thus, it is not necessary to manufacture imaging elements for respective image acquisition devices, thus preventing the manufacturing costs of imaging elements from increasing.
In the above-described aspect, it is preferred that the following conditional expression be satisfied.

$$0 < K \times Tan(\theta c) \leq P/2 \cdots (2)$$

Here, $\theta c$ is a chief-ray correction amount (angle) for the microlens at the position of a horizontal image height (C), and the position of the image height (C) is the point of intersection between the optical axis of the objective optical system and the imaging element. P is the pixel pitch in the imaging element, and K is the distance from surface apexes of the microlenses to the pixels (light-sensitive elements) in the imaging element.
By doing so, it is possible to suppress the occurrence of color shading, which is caused when a chief ray of the objective optical system enters pixels other than a target pixel.
In the above-described aspect, it is preferred that the following conditional expressions be satisfied.

$$P/2 \geq K \times (Tan(\theta H) - Tan(\alpha)) \cdots (3)$$

$$P/2 \geq K \times (Tan(\alpha) - Tan(\theta D)) \cdots (4)$$

Here, $\alpha$ is an angle formed by a chief ray at the horizontal maximum image height and the optical axis, $\theta H$ is a chief-ray correction amount (angle) for the microlens at the position of the horizontal maximum image height (H) from the center portion of the imaging element, $\theta D$ is a chief-ray correction amount (angle) for the microlens at the position of the horizontal image height (D) from the center portion of the imaging element, the position of the image height (D) is the point of intersection between a line drawn toward the center portion of the imaging element at an angle $\alpha$, with the optical axis of the objective optical system serving as an axis of symmetry, and the imaging element, P is the pixel pitch in the imaging element, and K is the distance from the surface apexes of the microlenses to the pixels (light-sensitive elements) in the imaging element.
By doing so, it is possible to suppress shading and crosstalk and to further suppress color mixing.
In the above-described aspect, it is preferred that the objective optical system include: two objective lenses that are arrayed in parallel and that form optical images of the subject in the imaging element; and a reflective member that is disposed between the objective lenses and the imaging element and that displaces the optical axis of the objective optical system.
By doing so, it is possible to allow oblique incidence in the optical system and to reduce the diameter of light flux in the prism, thus reducing the size of the image acquisition device. It is possible to simultaneously observe shading-suppressed images from the two optical systems and to allow stereoscopic observation by displaying the two images as a binocular image on a 3D monitor.
In the above-described aspect, it is preferred that the objective optical system include an optical-path splitting means; and that optical images of the subject obtained through splitting by the optical-path splitting means be formed in the imaging element as optical images with different focal positions.
By doing so, a reduction in size is allowed, and images with different focal positions can be acquired at the same time.

{Advantageous Effects of Invention}

According to the present invention, an advantageous effect is afforded in that it is possible to acquire, while suppressing shading and crosstalk, a plurality of images arrayed in parallel without shifting the microlenses of the imaging element and to allow a reduction in the size of the image acquisition device.

{Brief Description of Drawings}

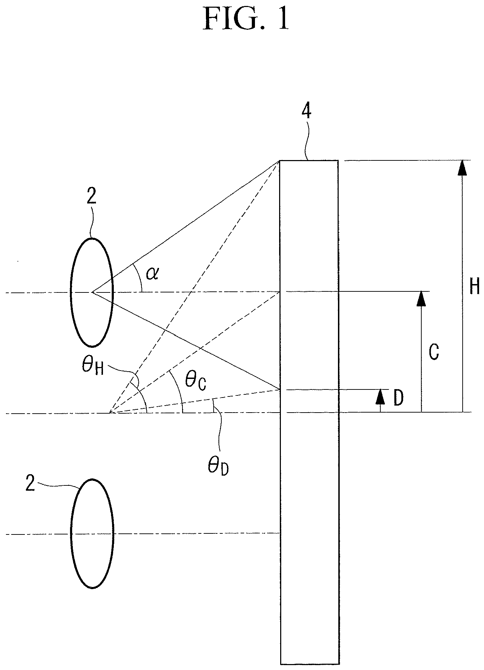

{Fig. 1} Fig. 1 is an explanatory diagram showing, in outline, the configuration of an image acquisition device according to an embodiment of the present invention.

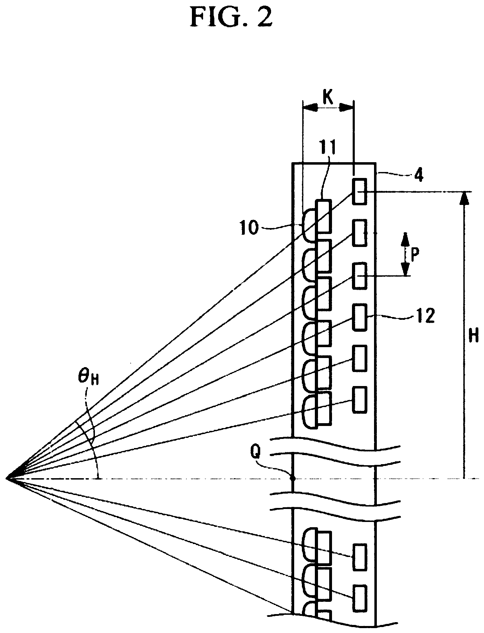

{Fig. 2} Fig. 2 is an explanatory diagram showing, in outline, the configuration of an imaging element applied to the image acquisition device according to the embodiment of the present invention.

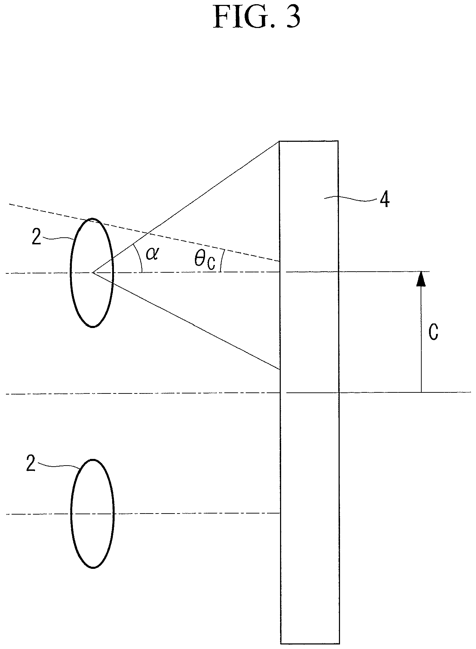

{Fig. 3} Fig. 3 is an explanatory diagram showing, in outline, the configuration of the image acquisition device according to the embodiment of the present invention.

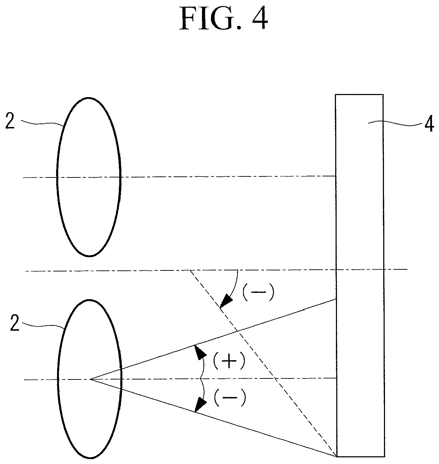

{Fig. 4} Fig. 4 is an explanatory diagram showing, in outline, the configuration of the image acquisition device according to the embodiment of the present invention.

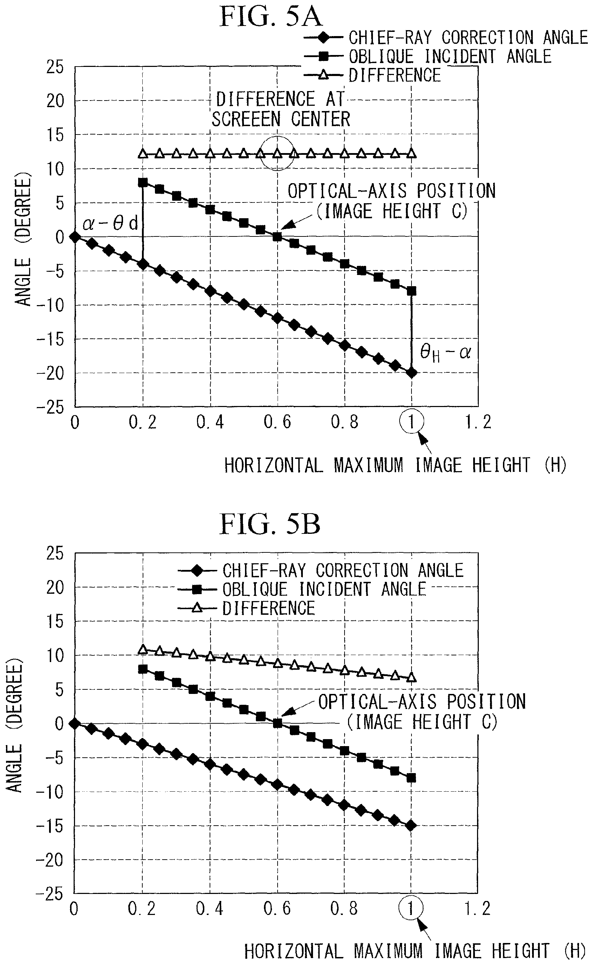

{Fig. 5A} Fig. 5A is a graph showing the relationship between chief-ray correction angles for microlenses and obliquely-incident-light angles in an optical system.

{Fig. 5B} Fig. 5B is a graph showing the relationship between the chief-ray correction angles for the microlenses and the obliquely-incident-light angles in the optical system.

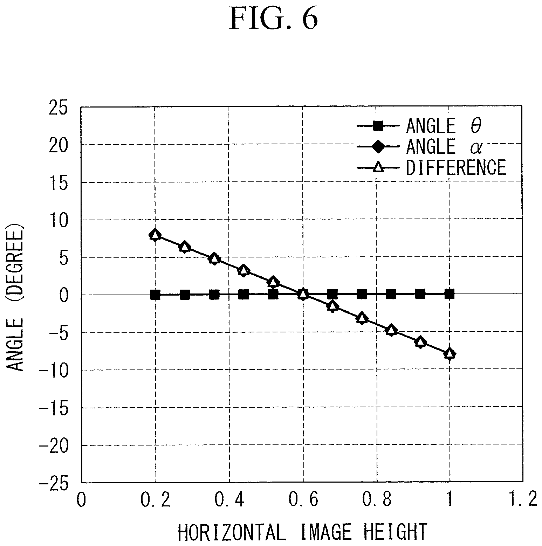

{Fig. 6} Fig. 6 is a graph showing the relationship between the chief-ray correction angles for the microlenses and the obliquely-incident-light angle in the optical system.

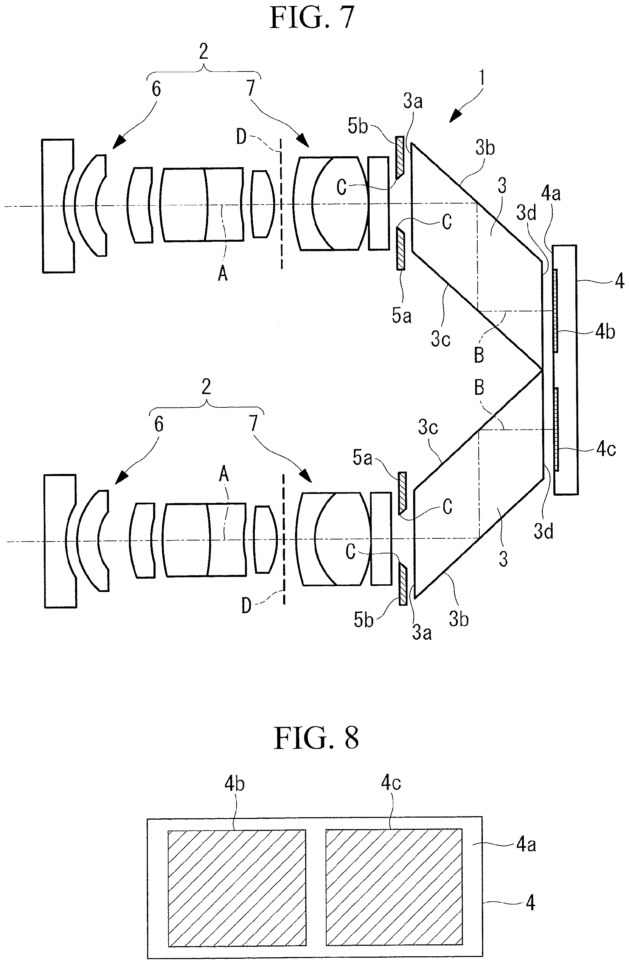

{Fig. 7} Fig. 7 is a sectional view showing the entire configuration of an image acquisition device according to Example 1 of the present invention.

{Fig. 8} Fig. 8 is an explanatory diagram of an imaging element applied to the image acquisition device according to Example 1 of the present invention.

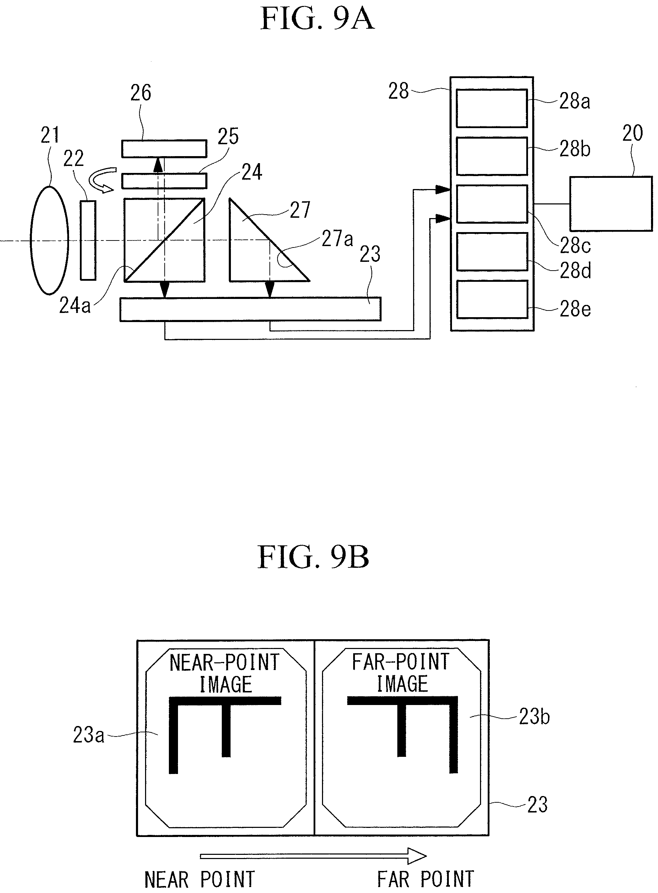

{Fig. 9A} Fig. 9A is a sectional view showing the entire configuration of an image acquisition system according to Example 2 of the present invention.

{Fig. 9B} Fig. 9B is a view showing orientations of a subject in images formed in first and second regions of the image acquisition system according to Example 2 of the present invention.

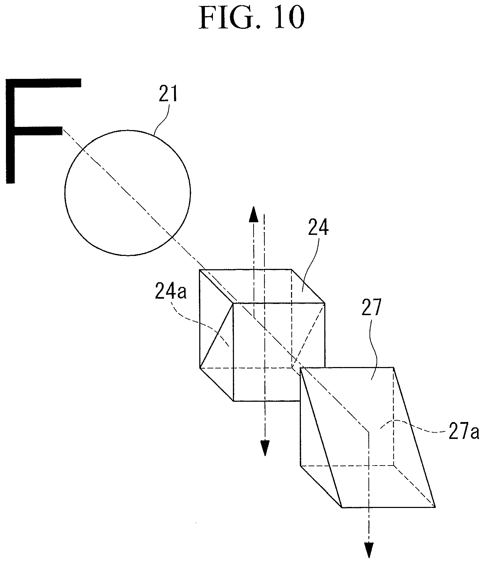

{Fig. 10} Fig. 10 is an explanatory diagram for images formed in imaging regions of the imaging element in the image acquisition system according to Example 2 of the present invention.

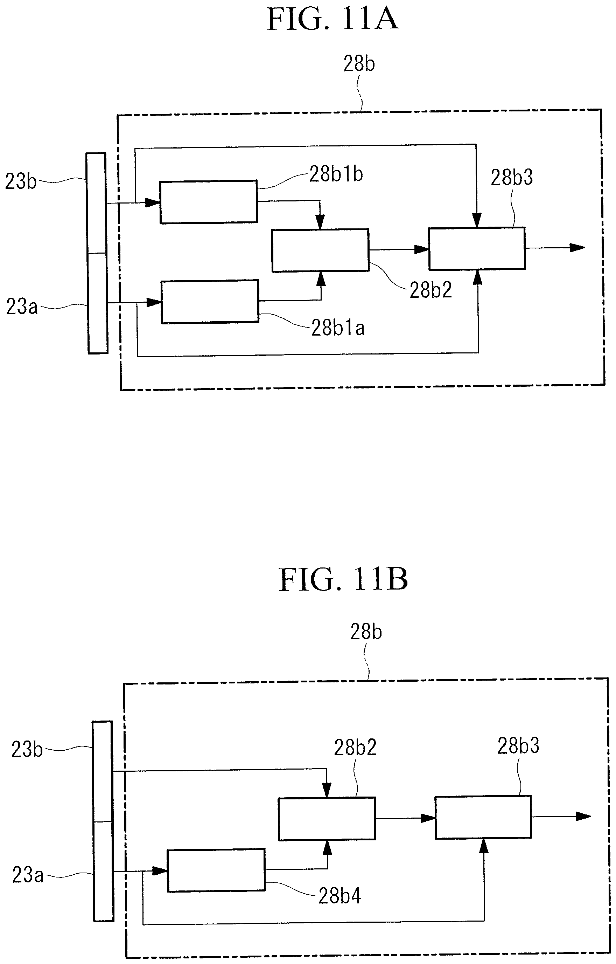

{Fig. 11A} Fig. 11A is a block diagram showing a configuration of an image processing unit in the image acquisition system according to Example 2 of the present invention.

{Fig. 11B} Fig. 11B is a block diagram showing another configuration of the image processing unit in the image acquisition system according to Example 2 of the present invention.

{Description of Embodiments}

An endoscope image-acquisition device 1 according to an embodiment of the present invention will be described below with reference to the drawings.

As shown in Fig. 1, the endoscope image-acquisition device 1 is provided with two objective optical systems 2 and an imaging element 4.

The two objective optical systems 2 are disposed in parallel, each focus light coming from a subject, and cause the focused light to be incident on a light-receiving surface of the imaging element. As shown in Fig. 2, the imaging element 4 has light-sensitive portions 12 that receive light from the objective optical systems 2 and also has a plurality of microlenses 10 and a plurality of color filters 11 that are arrayed at an entrance side of the light-sensitive portions 12, and a pixel is allocated to each of the microlenses 10.

The centers of the light-sensitive portions 12 at the pixels in the imaging element 4 are displaced with respect to the optical axes of the microlenses 10 such that the displacements therebetween are gradually increased in peripheral directions away from the center portion of the imaging element 4 toward peripheral portions thereof; and the position of the exit pupil of each objective optical system 2 is closer to an object than an imaging position of the objective optical system 2 is.

Incidentally, when imaging is performed by arraying, in parallel, imaging elements that are adjusted for the image-side telecentricity of an optical system, i.e., imaging elements that have no displacement between the center position of each microlens and the center of each light-sensitive portion (hereinafter, the displacement is referred to as "microlens correction amount"), and by using a binocular-stereoscopic-imaging optical system in which light is emitted from the exit pupil at a chief-ray angle $\alpha$, the difference between the chief-ray angle of an objective optical system and the microlens correction amount is a or $-\alpha$ in the horizontal direction, the difference in the horizontal direction is $2\alpha$, and the difference therebetween is increased (see Fig. 6).

On the other hand, as shown in Figs. 1 and 2, when $\alpha$ is the exit angle for a horizontal maximum image height H of the objective optical system, and $\theta$H is the microlens correction amount in the imaging element at that position, the difference between the correction amount and the exit angle can be expressed by

$\alpha$ - $\theta$H.

The difference between a correction amount $\theta$D for a microlens at a position in the opposite direction of the objective optical axis from the horizontal maximum image height H of the objective optical system and the chief-ray exit angle $\alpha$ of the objective optical system can be expressed by

$-\alpha$ - $\theta$D.

Here, when the differences between the chief-ray correction amounts for the microlenses and the chief-ray exit angles of the objective optical system are not equal in respective imaging ranges, the amounts of light entering adjacent pixels vary, thus causing shading. In other words, when the differences between the microlens correction amounts and the chief-ray angles from the exit pupil of the objective optical system are equal in the respective imaging ranges, shading does not occur.

Thus, the difference between the microlens correction amount and the chief-ray angle from the exit pupil of the objective optical system, for allowing shading to be suppressed, is

$$(\alpha - \theta H) - (-\alpha - \theta D) = 2\alpha - (\theta H - \theta D) \cdots (A).$$

Here, because $\theta$H is larger than $\theta$D, as shown in Fig. 1, the difference between the microlens correction amount and the chief-ray angle from the exit pupil of the objective optical system, for allowing shading to be suppressed, becomes smaller than $2\alpha$, which can be said to be a better condition than the above-described equation (A), thus allowing shading to be suppressed. Thus, it is desired that $\theta$H be finite, that is, the microlens correction amount be finite.

Then, when $\alpha - \theta H = -\alpha - \theta D$ is satisfied, it can be said that the differences between the microlens correction amounts and the chief-ray angles of the objective lens are equal, and thus shading does not occur.

Thus, $(\theta H - \theta D)/\alpha = 2$ is a desired condition in which shading does not occur.

On the other hand, because it is considered that a reduction in color reproduction and a reduction in resolving power at the center of the screen have a great influence on image quality, a configuration for suppressing crosstalk particularly at the center of the screen while completely suppressing crosstalk is desirable.

A condition for suppressing crosstalk particularly at the center of the screen while completely suppressing crosstalk will be discussed below.

As shown in Fig. 4, the chief-ray correction angle for a microlens and the obliquely-incident-light angle of the optical system are set to have negative signs in the direction opposite to the horizontal maximum image height from the central axis of the imaging element.

In this case, the chief-ray correction angle for the microlens and the obliquely-incident-light angle of the optical system are increased in the direction toward the negative side, as in the graphs shown in Figs. 5A and 5B.

An upper-most line shown in Figs. 5A and 5B indicates the difference between the above-described two angles (oblique incident angle - chief-ray correction amount). Fig. 5A is well-balanced so as to equalize the difference therebetween in the entire screen, by using the chief-ray correction angle (lower-most graph shown in Figs. 5A and 5B) as a parameter.

As described above, it is preferred that the endoscope image-acquisition device 1 of this embodiment be configured to satisfy the following conditional expression. Shading becomes large when $(\theta H - \theta D)/\alpha$ exceeds the upper limit of the following conditional expression or is lower than the lower limit thereof.

$$0.5 \leq (\theta H - \theta D)/\alpha \leq 3 \cdots (1)$$

Here, $\alpha$ is the angle formed by a chief ray at the horizontal maximum image height and the optical axis, $\theta$H is a chief-ray correction amount (angle) for the microlens at the position of the horizontal maximum image height (H) from the center portion of the imaging element, $\theta$D is a chief-ray correction amount (angle) for the microlens at the position of a horizontal image height (D) from the center portion of the imaging element, and the position of the image height (D) is the point of intersection between a line drawn toward the center portion of the imaging element at $\alpha$ degrees, with the optical axis of the objective optical system serving as an axis of symmetry, and the imaging element.

The endoscope image-acquisition device 1 is preferably configured so as to satisfy the following conditional expression.

$$0 < K \times \mathrm{Tan}(\theta c) \leq P/2 \cdots (2)$$

wherein $\theta c$ is a chief-ray correction amount (angle) for the microlens at the position of a horizontal image height (C), and the position of the image height (C) is the point of intersection between the optical axis of the objective optical system and the imaging element. P is a pixel pitch in the imaging element, and K is the distance from surface apexes of the microlenses to the pixels (the light-sensitive elements) in the imaging element.

When $K \times \tan(\theta c)$ becomes negative, this means that, as shown in Fig. 3, the microlens correction amount corrects a ray entering from the opposite direction from that in Fig. 1, with the optical axis serving as an axis of symmetry. In such an optical system in which the microlens correction amount $\theta c$ becomes negative, the exit pupil is positioned at the opposite side of the imaging surface from the object. In such an optical system, the optical system itself becomes large, and the image acquisition device itself becomes large.

When $K \times \tan(\theta c)$ becomes zero, the difference between the chief-ray angle of the objective optical system and the microlens correction amount is $\alpha$ or $-\alpha$ in the horizontal direction, the difference in the horizontal direction is $2\alpha$, and the difference therebetween is increased, as shown in Fig. 6. Thus, $k\tan(\theta c)$ is desirably set larger than 0°. When $K\tan(\theta c)$ exceeds P/2, the chief ray enters adjacent pixels, thus causing color shading.

Furthermore, the endoscope image-acquisition device 1 is configured to satisfy the following conditional expressions, thereby making it possible to suppress color mixing.

$$P/2 \geq K \times (\mathrm{Tan}(\theta H) - \mathrm{Tan}(\alpha)) \cdots (3)$$

$$P/2 \geq K \times (\mathrm{Tan}(\alpha) - \mathrm{Tan}(\theta D)) \cdots (4)$$

wherein $\alpha$ is the angle formed by the chief ray at the horizontal maximum image height and the optical axis, $\theta H$ is the chief-ray correction amount (angle) for the microlens at the position of the horizontal maximum image height (H) from the center portion of the imaging element, $\theta D$ is the chief-ray correction amount (angle) for the microlens at the position of the horizontal image height (D) from the center portion of the imaging element, the position of the image height (D) is the point of intersection between the line drawn toward the center portion of the imaging element at $\alpha$ degrees, with the optical axis of the objective optical system serving as an axis of symmetry, and the imaging element, P is the pixel pitch in the imaging element, and K is the distance from the surface apexes of the microlenses to the pixels (the light-sensitive elements) in the imaging element. When $K \times \mathrm{Tan}(\theta H) - K \times \mathrm{Tan}(\alpha)$ exceeds P/2, a chief ray emitted from the objective optical system 2 at the angle $\alpha$ enters adjacent pixels, thus causing color shading. Furthermore, when $K \times \mathrm{Tan}(\alpha) - K \times \mathrm{Tan}(\theta D)$ exceeds P/2, a chief ray emitted from the objective optical system 2 at the angle $-\alpha$ enters adjacent pixels, thus causing color shading.

Examples

Example 1

An endoscope image-acquisition device 1 according to an example 1 of the present invention will be described below with reference to the drawings.

Fig. 7 is a sectional view showing the entire configuration of the endoscope image-acquisition device 1 of this example. As shown in Fig. 7, the endoscope image-acquisition device 1 is provided with two objective optical systems 2 that are arrayed in parallel with a space therebetween, two parallelogram prisms 3 that are disposed at latter stages of the objective optical systems 2, one imaging element 4 that is disposed at a subsequent stage of the parallelogram prisms 3, and diaphragms 5a and 5b.

The two objective optical systems 2 are each provided with a first group 6 having a negative refractive power and a second group 7 having a positive refractive power, in this order from the object side. Light flux focused by the objective optical system 2 is reduced in diameter by the first group 6, is then expanded, and is focused again by the second group 7, thereby being imaged at the focal position thereof. The focal position of the second group 7 is made to match an imaging surface 4a of the imaging element 4, to be described later. The position of the exit pupil of the objective optical system 2 is designed so as to be closer to the object than the imaging element 4 is. The maximum chief-ray exit angle in the horizontal maximum image height H of the objective optical system 2 is 8 degrees (see Fig. 5A).

The parallelogram prisms 3 are each provided with a first surface 3a, a second surface 3b, a third surface 3c, and a fourth surface 3d. The first surface 3a is disposed so as to be perpendicular to an optical axis (incident optical axis) A of the objective optical system 2 so as to make light emitted from the second group 7 of the objective optical system 2 enter the first surface 3a, and the second surface 3b is disposed at an angle of 45 degrees with respect to the optical axis A of the objective optical system 2 so as to deflect light that has entered the parallelogram prism 3 from the first surface 3a. The third surface 3c is disposed parallel to the second surface 3b, and the fourth surface 3d is disposed parallel to the first surface 3a.

Light that has entered the parallelogram prism 3 from the first surface 3a along the incident optical axis A is deflected twice by the second surface 3b and by the third surface 3c and is then emitted from the fourth surface 3d toward the imaging element 4, which is disposed at the latter stage, along an exit optical axis B that is parallel to the incident optical axis A.

At this time, the two parallelogram prisms 3 are arrayed such that the space between the exit optical axes B becomes narrower than the space between the incident optical axes A, thereby making it possible to bring optical images, which are focused by the two objective optical systems 2 and which are imaged on the imaging surface 4a of the imaging element 4, close to each other and to reduce the size of the imaging surface 4a of the imaging element 4, which acquires two optical images at the same time.

The imaging element 4 is a CCD, for example, and, as shown in Fig. 8, two optical images focused by the objective optical systems 2 are formed in parallel in two effective light-receiving regions on the imaging surface 4a.

As shown in Fig. 2, the microlenses 10 and the color filters 11 are arrayed, for the respective pixels, at the object side of the light-sensitive portions (for example, photoelectric conversion elements) 12 in the imaging element 4.

The displacements between the center positions of the microlenses 10 and the centers of the light-sensitive portions 12 are increased in peripheral directions away from the center position Q of the imaging element 4. The displacement therebetween at the horizontal maximum-image-height position is 20 degrees, when it is expressed by the angle formed between a line connecting the center position of the microlens 10 with the center of the light-sensitive portion 12 and the optical axis of the objective optical system 2 (hereinafter, referred to as "microlens correction amount").

At this time, the differences between the microlens correction amounts and the chief-ray exit angles of the objective optical system 2 are calculated, as shown in Fig. 5A. By doing so, the differences between the microlens correction amounts and the chief-ray exit angles of the objective optical system 2 are equalized in imaging regions 4b and 4c, so that intensity/color shading does not occur. Thus, even one imaging element allows stereoscopic observation by forming binocular parallax images and allows observation with good image quality without causing shading.

Furthermore, when the position of the exit pupil of the objective optical system 2 is adjusted for the microlens correction amounts of the imaging element 4 (in the case of an image acquisition device shown in Fig. 2), shading does not occur. Thus, it is possible to directly apply this imaging element to an image acquisition device in which one image is formed by all pixels in the imaging element 4, as shown in Fig. 2. Furthermore, because it is also possible to apply this imaging element to an image acquisition device in which two images are formed in parallel, as in this example, it is not necessary to manufacture imaging elements in which microlens correction amounts are modified for respective image acquisition devices, thus preventing the manufacturing costs of imaging elements from increasing.

Because the positions of the exit pupils of the objective optical systems 2 are designed to be closer to the object than the imaging element 4 is, the prisms 3 can be reduced in size, and stereoscopic observation is allowed with a small image acquisition device.

Example 2

Figs. 9A and 9B are explanatory diagrams showing the configuration of an image acquisition device system according to an example 2 of the present invention: Fig. 9A is a diagram schematically showing the entire configuration thereof; and Fig. 9B is a diagram showing the orientations of a subject in images formed in first and second regions of the imaging element.

The image acquisition device system of this example includes an objective lens 21, a depolarizing plate 22, an imaging element 23, a polarizing beam splitter 24, a wave plate 25, a first reflective member 26, a second reflective member 27, and an image processing unit 28. The image acquisition device system is connected to an image display device 20, so that an image acquired by the image acquisition device system can be displayed on the image display device 20.

The objective lens 21 has a function of forming an image of light flux coming from an object and is configured so that the exit pupil is positioned closer to the object than the imaging element 23 is.

The depolarizing plate 22 is disposed between the objective lens 21 and the polarizing beam splitter 24. The imaging element 23 is formed of a rolling-shutter-type CMOS sensor and is disposed in the vicinity of the imaging position of the objective lens 21. In the imaging element 23, the centers of light-sensitive portions at respective pixels are displaced with respect to the optical axes of microlenses (not shown) in the imaging element 23 such that the displacements therebetween are gradually increased in peripheral directions away from the center portion of the imaging element 23 toward the peripheral portions thereof. Furthermore, the position of the exit pupil of the objective lens 21 is set closer to the object than the imaging element 23 is.

The polarizing beam splitter 24 is disposed in the light path between the objective lens 21 and the imaging element 23 and above a first region 23a of the imaging element 23 and splits, with a polarizing-beam-splitter surface 24a, the light flux coming from the objective lens 21 into reflected light flux and transmitted light flux. Here, it is assumed that the polarizing beam splitter 24 reflects linearly polarized light with an S-polarization component and transmits linearly polarized light with a P-polarization component.

The wave plate 25 is made of a $\lambda/4$ plate and is configured so as to be able to be rotated about the optical axis.

The first reflective member 26 is made of a mirror, reflects back the light flux that has been reflected by the polarizing-beam-splitter surface 24a and that has been transmitted through the wave plate 25.

The second reflective member 27 is made of a prism and reflects, at a total reflection surface 27a, light that has been transmitted through the polarizing beam splitter 4. The prism may have a total reflection surface by subjecting the total reflection surface 27a to mirror coating.

Then, the image acquisition device system of this example forms an image of light flux that has been reflected by the first reflective member 26 via the wave plate 25 and the polarizing beam splitter 24, in the first region 23a of the imaging element 23, and, meanwhile, forms an image of light flux that has been reflected by the second reflective member 27, in a second region 23b of the imaging element 23, the second region 23b being different from the first region 23a.

The image processing unit 28 is connected to the imaging element 23, is provided in a central processing unit (not shown), and has a first image processing unit 28a, a second image processing unit 28b, a third image processing unit 28c, a fourth image processing unit 28d, and a fifth image processing unit 28e.

The first image processing unit 28a is configured so as to correct the orientations (rotations) of an image in the first region 23a and an image in the second region 23b.

When a letter "F" shown in Fig. 10 is observed, for example, the orientations of images formed in the first region 23a and the second region 23b are shown in Fig. 9B. Specifically, the orientation of an image formed in the first region 23a is obtained by rotating the letter "F" about the center point of the first region 23a in a clockwise direction by 90 degrees and also rotating the letter "F" about a vertical axis in Fig. 9B that passes through the center point of the first region 23a by 180 degrees. The orientation of an image formed in the second region 23b is obtained by rotating the letter "F" about the center point of the second region 3b in a clockwise direction by 90 degrees.

Thus, when the images formed in the first region 23a and in the second region 23b are displayed on the image display device 20, the first image processing unit 28a rotates the images formed in the first region 23a and in the second region 23b about the center points of the respective regions in a counterclockwise direction by 90 degrees and further rotates the image in the first region 23a about the vertical axis, in Fig. 9B, which passes through the center point of the first region 23a, by 180 degrees, thereby correcting the mirrored images.

The third image processing unit 28c is configured so as to be capable of adjusting the white balance of the image in the first region 23a and that of the image in the second region 23b.

The fourth image processing unit 28d is configured so as to be capable of moving (selecting) the center position of the image in the first region 23a and that of the image in the second region 23b.

The fifth image processing unit 28e is configured so as to be capable of adjusting the display range (magnification) of the image in the first region 23a and that of the image in the second region 23b.

The second image processing unit 28b corresponds to an image selecting unit of the present invention and is configured so as to compare the image in the first region 23a with the image in the second region 23b and select the image in the focused region as an image to be displayed.

Specifically, as shown in Fig. 11A, for example, the second image processing unit 28b has high-pass filters 28b1a and 28b1b that are connected to the regions 23a and 23b, respectively, a comparator 28b2 that is connected to the high-pass filters 28b1a and 28b1b, and a switch 28b3 that is connected to the comparator 28b2 and to the regions 23a and 23b and is configured such that the high-pass filters 28b1a and 28b1b extract high-frequency components from the images in the first region 23a and the second region 23b, the comparator 28b2 compares the extracted high-frequency components, and the switch 28b3 selects the image in the region that has more high-frequency components.

For example, as shown in Fig. 11B, the second image processing unit 28b may have a defocusing filter 28b4 that is connected only to one region 23a, a comparator 28b2 that is connected to the defocusing filter 28b4 and that is also connected to the other region 23b, and a switch 28b3 that is connected to the region 23a and the comparator 28b2 and may be configured such that the comparator 28b2 compares an image signal in the region 23a that is defocused by the defocusing filter 28b4 with an image signal in the region 23b that is not defocused, and the switch 28b3 selects the image in the region 23b for a matched portion and the image in the region 23a for an unmatched portion.

The image display device 20 has a display region for displaying an image selected by the second image processing unit 28b. The image display device 20 may have display regions for displaying images formed in the first and second regions 23a and 23b.

In the thus-configured image acquisition device system, light flux from the objective lens 21 passes through the depolarizing plate 22, thus being depolarized in the polarization direction, and then enters the polarizing beam splitter 24. The light entering the polarizing beam splitter 24 is split by the polarizing-beam-splitter surface 24a into linearly polarized light with an S-polarization component and linearly polarized light with a P-polarization component.

The light flux of linearly polarized light with the S-polarization component, which is reflected by the polarizing-beam-splitter surface 24a, passes through the $\lambda/4$ plate 25, thus converting the polarization state thereof into circularly polarized light, and is reflected by the mirror 26. The light flux reflected by the mirror 26 passes through the $\lambda/4$ plate 25 again, thus converting the polarization state thereof from circularly polarized light into linearly polarized light with the P-polarization component, enters the polarizing beam splitter 24 again, is transmitted through the polarizing-beam-splitter surface 24a, and forms an image in the first region 23a of the CMOS sensor 23.

The light flux of linearly polarized light with the P-polarization component that has passed through the objective lens 21 and the depolarizing plate 22 and has been transmitted through the polarizing-beam-splitter surface 4a when entering the polarizing beam splitter 24 is reflected by the total reflection surface 27a of the prism 27 and forms an image in the second region 23b of the CMOS sensor 23.

The CMOS sensor 23 (imaging element) is of the rolling shutter type, as described above, and reads an image on a line basis in the direction indicated by the arrow in Fig. 9B.

The second image processing unit 28b compares the images formed in the first region 23a and the second region 23b, the images being read on a line basis, and selects focused images as an image to be displayed.

The line-based images selected by the second image processing unit 28b are combined and displayed on the image display device 20.

According to the image acquisition device system of this example, because the polarizing beam splitter 24 is used as a splitting element, and the wave plate 25 is used, the polarization direction of the light flux that has been reflected by the polarizing beam splitter 24 is changed by 90 degrees via the wave plate 25, thereby making it possible to maintain the brightness of the two images formed in the first region 23a and the second region 23b almost equal. Then, because the $\lambda/4$ plate 25 is used as the wave plate, the light flux that has been reflected by the polarizing beam splitter 24 is returned by the first reflective member 26, thereby being changed by 90 degrees in the polarization direction and thereby making it possible to efficiently form an image of the light flux in the imaging element 23 with little loss of the brightness of the light flux.

In a case in which the brightness of two images formed in the first region 23a and the second region 23b does not need to be taken into account, a half mirror may be used as the splitting element 24.

According to the image acquisition device system of this example, the second image processing unit 28b, which serves as an image selecting unit, compares the images formed in the first region 23a and the second region 23b and selects the image in the focused region as an image to be displayed; therefore, it is possible to acquire an image with a deep focal depth in a continuous range and to observe, on the image display device 20, an image to be observed with a deep depth of field in a wide continuous range.

According to the image acquisition device system of this example, the first image processing unit 28a is provided, thus allowing the orientations (rotations) of the two images formed in the first region 23a and the second region 23b to be adjusted; therefore, it is possible to make the two images have the same orientations for display. The first image processing unit 28a can correct the rotation amounts of the two images, which are caused by manufacturing errors in the prism etc.; therefore, it is not necessary to provide a mechanical prism-adjusting mechanism or the like for correcting the image orientation. Thus, it is possible to reduce the entire size of the image acquisition device and to reduce the manufacturing costs.

Because the third image processing unit 28c is provided, when a difference in color between the two images formed in the first region 23a and the second region 23b occurs due to a coating manufacturing error in the optical system, the white balance of the two images can be adjusted.

Furthermore, because the fourth image processing unit 28d and the fifth image processing unit 28e are provided, when the center positions and the magnifications of the two images formed in the first region 23a and the second region 23b do not match due to an assembly error or a manufacturing error in the prism 27 and the polarizing beam splitter 24, it is possible to correct the mismatched positions and the mismatched magnifications of the two images by adjusting the center positions and the display ranges.

It is more preferable to provide an image processing unit (not shown) that is capable of adjusting the speed of an electronic shutter for images formed in the first region 23a and the second region 23b. By doing so, the brightness of the two images formed in the first region 23a and the second region 23b can be adjusted by adjusting the speed of the electronic shutter.

Because the $\lambda/4$ plate 25 is configured so as to be able to be rotated about the optical axis, it is possible to adjust the polarization state of light flux by rotating the $\lambda/4$ plate 25 and to adjust the amount of light that is transmitted through the polarizing beam splitter 24 and that enters the first region 23a. Thus, the difference in brightness between the two images formed in the first region 23a and the second region 23b, which is caused by a manufacturing error etc. in the optical system, can be easily adjusted.

The position of the exit pupil of the objective lens 21 is set closer to the object than the imaging element is, thus reducing the objective lens 21 and also reducing the height of rays entering the prism; therefore, the prism 27 and the polarizing beam splitter 24 are reduced in size, thus making it possible to realize a compact image acquisition device that has different focal positions.

The position of the exit pupil of the objective lens 21 is set closer to the object than the imaging element is, and the CMOS sensor 23 is configured such that the displacements of the centers of the light-sensitive portions at the pixels with respect to the optical axes of the microlenses are gradually increased in peripheral directions away from the center portion of the CMOS sensor toward the peripheral portions thereof. Thus, it is possible to realize an image acquisition device that produces little shading and that produces a wide depth.

In a case in which the light flux that has passed through the objective lens 21 from the subject has a polarization component biased in the polarization direction (for example, only P-polarization component), if the depolarizing plate 22 is not provided, the amount of light flux that is reflected by the polarizing beam splitter 24 is widely different from the amount of light flux that is transmitted therethrough. As a result, when the image selecting unit 28a2 compares two images formed in the first region 23a and the second region 23b, selects focused images on a line basis, and combines the selected line-based images into the entire image, there is a possibility that the brightness of the combined entire image varies for each line, thus posing a problem for observation.

In this way, according to the image acquisition device system of this example, because the depolarizing plate 22 is provided, even when the light flux passing through the objective lens 21 has a polarization component biased in the polarization direction, the light flux is made to pass through the depolarizing plate 22, thereby making it possible to randomize the polarization direction of the light flux, thus maintaining the brightness of the two images formed in the first region 23a and the second region 23b almost equal. As a result, when the image selecting unit 28b compares the two images formed in the first region 23a and the second region 23b, selects focused images on a line basis, and combines the selected line-based images into an entire image, it is possible to acquire an entire image with a uniform brightness over the respective lines.

{Reference Signs List}

2 objective lens
4 imaging element
10 microlens
11 color filter
12 light-sensitive portion

**Claims**

1. An endoscope image-acquisition device (1) comprising:

an objective optical system (2) that focuses light from a subject to form two images in parallel; and
an imaging element (4) that has a plurality of microlenses (10) arrayed at an entrance side of light coming from the objective optical system (2) and in which pixels are allocated to the respective microlenses (10),
wherein the two images of the subject focused by the objective optical system (2) are formed in parallel in two effective light-receiving regions on the imaging element (4),
wherein the center portions of the two effective light-receiving regions define a first axis;
wherein the centers of light-sensitive portions (12) at the pixels in the imaging element (4) are displaced with respect to the optical axes of the microlenses (10) such that displacements therebetween are gradually increased in peripheral directions away from the center portion of the imaging element (4) toward peripheral portions thereof;
the position of an exit pupil of the objective optical system (2) is closer to an object than an imaging position of the objective optical system (2) is;
**characterized in that**
the peripheral directions in which the displacements are gradually increased also include directions along the first axis, wherein the following conditional expression is satisfied:

$$0.5 \leq (\theta H - \theta D)/\alpha \leq 3 \cdots (1)$$

where $\alpha$ is an angle formed by a chief ray of the objective optical system (2) at a horizontal maximum image height (H) and an optical axis, $\theta H$ is a chief-ray correction amount angle for the microlens (10) at the horizontal maximum image height (H) from the center portion of the imaging element (4), $\theta D$ is a chief-ray correction amount angle for the microlens (10) at a distance (D) from the center portion of the imaging element (4), the position of an image height at the distance (D) being the point of intersection between a line drawn toward the center portion of the imaging element (4) at an angle $\alpha$, with the optical axis of the objective optical system (2) serving as an axis of symmetry, and the imaging element (4),

wherein each of the chief-ray correction amount angle for the microlens (10) at the horizontal maximum image height (H) and the chief-ray correction amount angle for the microlens (10) at the distance (D) is an angle formed between a line connecting a center position of the microlens (10) with the center of the respective light-sensitive portion (12) and the optical axis of the objective optical system (2).

2. An endoscope image-acquisition device (1) according to claim 1, wherein the following conditional expression is satisfied:

$$0 < K \times Tan(\theta c) \leq P/2 \cdots (2)$$

where, $\theta c$ is a chief-ray correction amount (angle) for the microlens (10) at the position of a horizontal image height (C), the position of the image height (C) is the point of intersection between the optical axis of the objective optical system (2) and the imaging element (4), P is the pixel pitch in the imaging element (4), and K is the distance from surface apexes of the microlenses (10) to the pixels (light-sensitive elements) in the imaging element (4).

3. An endoscope image-acquisition device (1) according to claim 2, wherein the following conditional expressions are satisfied:

$$P/2 \geq K \times (Tan(\theta H) - Tan(\alpha)) \cdots (3)$$

$$P/2 \geq K \times (Tan(\alpha) - Tan(\theta D)) \cdots (4)$$

4. An endoscope image-acquisition device (1) according to one of claims 1 to 3, wherein the objective optical system (2) includes:

two objective lenses that are arrayed in parallel and that form optical images of the subject in the imaging element (4); and
a reflective member that is disposed between the objective lenses and the imaging element (4) and that displaces the optical axis of the objective optical system (2).

5. An endoscope image-acquisition device (1) according to one of claims 1 to 3,
wherein the objective optical system (2) includes an optical-path splitting means (24); and
optical images of the subject obtained through splitting by the optical-path splitting means (24) are formed in the imaging element (4) as optical images with different focal positions.

**Patentansprüche**

1. Endoskopbild-Erfassungsvorrichtung (1), umfassend:

ein optisches Objektivsystem (2), das Licht von einem Subjekt fokussiert, um parallel zwei Bilder zu bilden; und
ein Abbildungselement (4), das eine Mehrzahl von Mikrolinsen (10) aufweist, die an einer Eintrittsseite von vom optischen Objektivsystem (2) kommendem Licht angeordnet ist und in der Pixel den jeweiligen Mikrolinsen (10) zugeordnet sind,

wobei die zwei Bilder des vom optischen Objektivsystem (2) fokussierten Subjekts parallel in zwei effektiven Lichtempfangsbereichen auf dem Abbildungselement (4) gebildet werden,

wobei die Mittelabschnitte der zwei effektiven Lichtempfangsbereiche eine erste Achse definieren,

wobei die Mittelpunkte von lichtempfindlichen Abschnitten (12) an den Pixeln im Abbildungselement (4) so in Bezug auf die optischen Achsen der Mikrolinsen (10) versetzt sind, dass Abstände zwischen diesen in peripheren Richtungen weg vom Mittelabschnitt des Abbildungselements (4) zu peripheren Abschnitten davon allmählich ansteigen;

sich die Position einer Austrittspupille des optischen Objektivsystems (2) näher an einem Objekt als an einer Abbildungsposition des optischen Objektivsystems (2) befindet;

**dadurch gekennzeichnet, dass**

die peripheren Richtungen, in die die Abstände allmählich ansteigen, ebenfalls Richtungen entlang der ersten Achse enthalten, wobei der folgende bedingte Ausdruck erfüllt ist:

$$0{,}5 \leq (\theta H - \theta D)/\alpha \leq 3 \ \dots \ (1)$$

wobei $\alpha$ ein von einem Hauptstrahl des optischen Objektivsystems (2) bei einer maximalen horizontalen Bildgröße (H) und einer optischen Achse gebildeter Winkel ist, $\theta H$ ein Hauptstrahlkorrekturwinkelbetrag für die Mikrolinse (10) an der maximalen horizontalen Bildgröße (H) vom Mittelabschnitt des Abbildungselements (4) ist, $\theta D$ ein Hauptstrahlkorrekturwinkelbetrag für die Mikrolinse (10) in einer Distanz (D) vom Mittelabschnitt des Abbildungselements (4) ist, wobei die Position einer Bildhöhe in der Distanz (D) der Schnittpunkt zwischen einer zum Mittelabschnitt des Abbildungselements (4) in einem Winkel $\alpha$ gezogenen Linie mit der optischen Achse des optischen Objektivsystems (2) als Symmetrieachse und dem Abbildungselement (4) ist,

wobei der Hauptstrahlkorrekturwinkelbetrag für die Mikrolinse (10) an der maximalen horizontalen Bildhöhe (H) bzw. der Hauptstrahlkorrekturwinkelbetrag für die Mikrolinse (10) in der Distanz (D) jeweils ein Winkel ist, der zwischen einer Linie, die eine Mittelposition der Mikrolinse (10) mit dem Mittelpunkt des jeweiligen lichtempfindlichen Abschnitts (12) verbindet, und der optischen Achse des optischen Objektivsystems (2) gebildet ist.

2. Endoskopbild-Erfassungsvorrichtung (1) nach Anspruch 1, wobei der folgende bedingte Ausdruck erfüllt ist:

$$0 < K \times Tan(\theta c) \leq P/2 \ \dots \ (2),$$

wobei $\theta c$ ein Hauptstrahlkorrekturbetrag (WinKel) für die Mikrolinse (10) an der Position einer horizontalen Bildhöhe (C) ist, wobei die Position der Bildhöhe (C) der Schnittpunkt zwischen der optischen Achse des optischen Objektivsystems (2) und dem Abbildungselement (4) ist, P der Pixelabstand im Abbildungselement (4) ist und K die Distanz von Oberflächenapizes der Mikrolinsen (10) zu den Pixeln (lichtempfindlichen Elementen) im Abbildungselement (4) ist.

3. Endoskopbild-Erfassungsvorrichtung (1) nach Anspruch 2, wobei die folgenden bedingten Ausdrücke erfüllt sind:

$$P/2 \geq K \times (Tan(\theta H) - Tan(\alpha)) \ \dots \ (3)$$

$$P/2 \geq K \times (Tan(\alpha) - Tan(\theta D)) \ \dots \ (4).$$

4. Endoskopbild-Erfassungsvorrichtung (1) nach einem der Ansprüche 1 bis 3, wobei das optische Objektivsystem (2) enthält:

zwei Objektivlinsen, die parallel angeordnet sind und die optische Bilder des Subjekts im Abbildungselement (4) bilden; und

ein Spiegelelement, das zwischen den Objektivlinsen und dem Abbildungselement (4) angeordnet ist und das die optische Achse des optischen Objektivsystems (2) versetzt.

5. Endoskopbild-Erfassungsvorrichtung (1) nach einem der Ansprüche 1 bis 3, wobei das optische Objektivsystem (2) Mittel zum Teilen des optischen Pfads (24) enthält; und
optische Bilder des Subjekts, die durch Teilen durch das Mittel zum Teilen des optischen Pfads (24) erhalten werden, im Abbildungselement (4) als optische Bilder mit unterschiedlichen Fokuslagen gebildet werden.

## Revendications

1.  Dispositif d'acquisition d'image d'endoscope (1) comprenant :

    un système optique d'objectif (2) qui focalise une lumière provenant d'un sujet pour former deux images en parallèle ; et
    un élément d'imagerie (4) qui présente une pluralité de microlentilles (10) agencées en réseau au niveau d'un côté d'entrée d'une lumière provenant du système optique d'objectif (2) et dans lequel des pixels sont affectés aux microlentilles respectives (10),
    les deux images du sujet focalisé par le système optique d'objectif (2) étant formées en parallèle dans deux régions de réception de lumière effectives sur l'élément d'imagerie (4),
    les parties centrales des deux régions de réception de lumière effectives définissant un premier axe ;
    les centres de parties sensibles à la lumière (12) au niveau des pixels dans l'élément d'imagerie (4) étant déplacés par rapport aux axes optiques des microlentilles (10) de telle sorte que des déplacements entre ceux-ci sont graduellement accrus dans des directions périphériques à l'opposé de la partie centrale de l'élément d'imagerie (4) vers des parties périphériques de celui-ci ;
    la position d'une pupille de sortie du système optique d'objectif (2) étant plus proche d'un objet que ne l'est une position d'imagerie du système optique d'objectif (2) ;
    **caractérisé par le fait que**
    les directions périphériques dans lesquelles les déplacements sont graduellement accrus comprennent également des directions le long du premier axe, l'expression conditionnelle suivante étant satisfaite :

    $$0,5 \leq (\theta H - \theta D)/\alpha \leq 3 \ ... \ (1)$$

    où a est un angle formé par un rayon principal du système optique d'objectif (2) à une hauteur d'image maximale horizontale (H) et un axe optique, $\theta H$ est un angle de quantité de correction de rayon principal pour la microlentille (10) à la hauteur d'image maximale horizontale (H) depuis la partie centrale de l'élément d'imagerie (4), $\theta D$ est un angle de quantité de correction de rayon principal pour la microlentille (10) à une distance (D) depuis la partie centrale de l'élément d'imagerie (4), la position d'une hauteur d'image à la distance (D) étant le point d'intersection entre une ligne tirée vers la partie centrale de l'élément d'imagerie (4) à un angle $\alpha$, avec l'axe optique du système optique d'objectif (2) servant en tant qu'axe de symétrie, et l'élément d'imagerie (4),
    chacun de l'angle de quantité de correction de rayon principal pour la microlentille (10) à la hauteur d'image maximale horizontale (H) et de l'angle de quantité de correction de rayon principal pour la microlentille (10) à la distance (D) étant un angle formé entre une ligne reliant une position centrale de la microlentille (10) avec le centre de la partie sensible à la lumière respective (12) et l'axe optique du système optique d'objectif (2).

2.  Dispositif d'acquisition d'image d'endoscope (1) selon la revendication 1, dans lequel l'expression conditionnelle suivante est satisfaite :

    $$0 < K \times Tan(\theta c) \leq P/2 \ ... \ (2)$$

    où $\theta c$ est une quantité de correction de rayon principal (angle) pour la microlentille (10) au niveau de la position d'une hauteur d'image horizontale (C), la position de la hauteur d'image (C) est le point d'intersection entre l'axe optique du système optique d'objectif (2) et l'élément d'imagerie (4), P est le pas de pixel dans l'élément d'imagerie (4), et K est la distance depuis des sommets de surface des microlentilles (10) vers les pixels (éléments sensibles à la lumière) dans l'élément d'imagerie (4).

3.  Dispositif d'acquisition d'image d'endoscope (1) selon la revendication 2, dans lequel les expressions conditionnelles suivantes sont satisfaites :

$$P/2 \geq K \times (\mathrm{Tan}(\theta H) - \mathrm{Tan}(\alpha)) \ldots (3)$$

$$P/2 \geq K \times (\mathrm{Tan}(\alpha) - \mathrm{Tan}(\theta D)) \ldots (4).$$

4. Dispositif d'acquisition d'image d'endoscope (1) selon l'une des revendications 1 à 3, dans lequel le système optique d'objectif (2) comprend :

deux objectifs qui sont agencés en réseau en parallèle et qui forment des images optiques du sujet dans l'élément d'imagerie (4) ; et
un élément réfléchissant qui est disposé entre les objectifs et l'élément d'imagerie (4) et qui déplace l'axe optique du système optique d'objectif (2).

5. Dispositif d'acquisition d'image d'endoscope (1) selon l'une des revendications 1 à 3, dans lequel le système optique d'objectif (2) comprend des moyens de division de chemin optique (24) ; et
des images optiques du sujet obtenues par l'intermédiaire d'une division par les moyens de division de chemin optique (24) sont formées dans l'élément d'imagerie (4) en tant qu'images optiques ayant différentes positions focales.

# FIG. 1

# FIG. 2

# FIG. 3

FIG. 4

## FIG. 5A

## FIG. 5B

# FIG. 6

# FIG. 7

# FIG. 8

# FIG. 9A

# FIG. 9B

# FIG. 10

# FIG. 11A

# FIG. 11B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI05346556 B
- JP 2010056345 A

- JP 4054094 B